# EUROPEAN PATENT APPLICATION

(11) **EP 2 876 487 A1**
(43) Date of publication of application: **27.05.2015**
(21) Application number: 14194384.5
(22) Date of filing: 21.11.2014
(51) Int. Cl.: G02C 7/04, A61B 3/16

(54) **Ophthalmic lens with intraocular pressure monitoring system**

(30) Priority: 22.11.2013 US 201314087217
(71) Applicant: Johnson & Johnson Vision Care, Inc., Jacksonville, FL 32256 (US)
(72) Inventor: Pugh, Randall Braxton, St. Johns, FL 32259 (US)
(74) Representative: Carpmaels & Ransford LLP

(57) **Abstract**

The present invention relates to an ophthalmic device with an intraocular pressure monitoring system and associated methods. In some embodiments, the ophthalmic device can be a contact lens with an intraocular pressure monitoring system that is not dependent on eye ball shape or change over time. Further, the intraocular pressure monitoring system may include elements for delivering audible and/or visual messages to the user that can be useful for the monitoring and treatment of glaucoma. The audible and/or visual messages can be signals communicated to the user using one or both of the ophthalmic device and a wireless device in communication with the ophthalmic device.

## Description

### FIELD OF THE INVENTION

The present invention relates to an energized ophthalmic device with an intraocular pressure monitoring system, and more specifically, an intraocular pressure monitoring system that is not dependent on eye ball shape or change over time.

### BACKGROUND OF THE INVENTION

Traditionally, an ophthalmic device, such as a contact lens, an intraocular lens, or a punctal plug, included a biocompatible device with a corrective, cosmetic, or therapeutic quality. A contact lens, for example, may provide one or more of vision correcting functionality, cosmetic enhancement, and therapeutic effects. Each function is provided by a physical characteristic of the lens. A design incorporating a refractive quality into a lens may provide a vision corrective function. A pigment incorporated into the lens may provide a cosmetic enhancement. An active agent incorporated into a lens may provide a therapeutic functionality. Such physical characteristics are accomplished without the lens entering into an energized state. An ophthalmic device has traditionally been a passive device.

Novel ophthalmic devices based on energized ophthalmic inserts have recently been described. These devices may use the energization function to power active optical components. For example, a wearable lens may incorporate a lens assembly having an electronically adjustable focus to augment or enhance performance of the eye.

Moreover, as electronic devices continue to be miniaturized, it is becoming increasingly more likely to create wearable or embeddable microelectronic devices for a variety of uses that can help with the diagnosis and treatment eye related conditions. One condition that currently affects an increasing number of people is glaucoma. Glaucoma is a debilitating intraocular pressure-associated optic neuropathy disease that can permanently damage vision and lead to blindness if left untreated. Early diagnosis and treatment is therefore desired. However, because the loss of vision associated with glaucoma occurs gradually over a long period of time, symptoms are hard to detect without actual testing until the disease is quite advanced.

Diagnosis of glaucoma is performed as part of eye examinations by eye care practitioners. Testing for glaucoma includes measuring the intraocular pressure of a patient's eye. Tonometry (inner eye pressure via puff test), ophthalmoscopy (dilated eye exam to look at the shape and color of the optic nerve), perimetry (visual field test), gonioscopy (test to determine the angle in the eye where the iris meets the cornea), pachymetry (determine cornea thickness), and nerve fiber analysis (determines the thickness of the nerve fiber layer) are all tests performed to diagnose a patient with glaucoma. Some of the aforementioned tests are more complex than others and all require special equipment. As a result, most patients are usually diagnosed using tonometry to measure intraocular pressure and treat when the intraocular pressure is above a normal level. Most treatments can include using medications that must be administered for the rest of a patient's life.

Intraocular pressure varies due to a number of factors both throughout the day and night. Diurnal factors can affect the intraocular pressure of a patient and therefore the diagnosis of glaucoma. In some cases, due to these changes, a person can be misdiagnosed by a single test that causes him/her to use these medications for the remainder of his/her life. The factors that can affect intraocular pressure readings include exercise, fluid intake, caffeine, systemic medications, respiration and heart rate, glycerol consumption, and other everyday medications. Consequently, new devices that can be used to monitor intraocular pressure at various points throughout the day/conditions are desired.

In an effort to provide a device that can be used to monitor the intraocular pressure of a patient's eye in simple manners, a device with a strain gage that can be placed on the eye has been recently described. Although this device may provide a change of shape and/or pressure, the accuracy can be compromised due to tear film consistency changes. In order to provide an accurate measurement that would account for tear film changes, continuous calibration of the strain gage/device would be required. As a result, a more accurate, practical and reliable device that can monitor changes in a patient's intraocular pressure innocuously and without delay is desired.

### SUMMARY OF THE INVENTION

The foregoing needs are met, to a great extent, by the present invention, wherein in one aspect an energized ophthalmic device incorporating an intraocular pressure monitoring system is disclosed. The intraocular pressure monitoring system can include a micro-piezoelectric element with a feedback circuit that can be used to measure intraocular pressure by outputting a signal and analyzing the change in the signal that returns to the feedback circuit relating to the intraocular pressure of a patient's eye.

According to some aspects of the disclosure, an ophthalmic device including an intraocular pressure monitoring system is disclosed. The ophthalmic device comprising: a media insert comprising a front curve arcuate surface and a back curve arcuate surface. The front curve arcuate surface and the back curve arcuate surface form a cavity capable of containing an energy source dimensioned to conform to an area within the cavity. The energy source being in electrical connection and capable of energizing a micro-piezoelectric element with an electronic feedback circuit and a controller, the controller comprising a computer processor in digital communication with a digital media storage device and wherein the digital media storage device stores software code, and a transmitter in logical communication with the processor and also in logical communication with a communication network. The software being executable upon demand and operative with the processor to: output and detect the change of a signal using the micro-piezoelectric element with the electronic feedback circuit; receive through the communication network from the feedback circuit the change of said outputted signal; and determine the intraocular pressure of a user's eye using the change of said outputted signal.

In additional aspects of the disclosure, a method of monitoring the intraocular pressure of a patient's eye is disclosed. The method comprising: providing an ophthalmic device with a intraocular pressure monitoring system comprising an energy source in electrical connection and capable of energizing a micro-piezoelectric element with an electronic feedback circuit and a controller comprising a computer processor, a digital media storage device, a transmitter in logical communication with the processor and also in logical communication with a communication network; outputting and detecting the change of a signal using the micro-piezoelectric element with the electronic feedback circuit; receiving through the communication network from the feedback circuit the change of said outputted signal; and determining the intraocular pressure of a user's eye using the change of said outputted signal.

### BRIEF DESCRIPTION OF THE DRAWINGS

The foregoing and other features and advantages of the invention will be apparent from the following, more particular description of preferred embodiments of the invention, as illustrated in the accompanying drawings.
Fig. 1A is a diagrammatic cross section representation of a first exemplary energized ophthalmic device comprising both optics and an intraocular pressure monitoring system in accordance with aspects of the present disclosure;
Fig. 1B is an enlarged portion of the cross section depicted in Fig. 1A showing aspects of the intraocular pressure monitoring system in accordance with aspects of the present disclosure;
Fig. 2A is a diagrammatic representation of the top view of a media insert that may be included as part of an ophthalmic device comprising both optics and the intraocular pressure monitoring system in accordance with aspects of the present disclosure;
Fig. 2B is a diagrammatic representation of an isometric view of an ophthalmic device including the media insert depicted in Fig. 2A comprising both optics and the intraocular pressure monitoring system in accordance with aspects of the present disclosure;
Fig. 3 is a diagrammatic representation of another exemplary energized ophthalmic device comprising both optics and the intraocular pressure monitoring system in accordance with aspects of the present disclosure;
Fig. 4 is a schematic diagram of an exemplary cross section of a stacked die integrated components implementing the intraocular pressure monitoring system in accordance with aspects of the present disclosure;
Fig. 5 is a schematic diagram of a processor that may be used to implement some aspects of the present disclosure; and
Fig. 6 illustrates exemplary method steps that may be used to implement the intraocular pressure monitoring system of the ophthalmic device according to aspects of the present disclosure.

### DETAILED DESCRIPTION OF THE INVENTION

The disclosure will now be described with reference to the drawing figures, in which like reference numerals refer to like parts throughout.

Various aspects of the ophthalmic device and method disclosed may be illustrated by describing components that are coupled, sealed, attached, and/or joined together. As used herein, the terms "coupled," "sealed," "attached," and/or "joined" are used to indicate either a direct connection between two components or, where appropriate, an indirect connection to one another through intervening or intermediate components. In contrast, when a component is referred to as being "directly coupled," "directly sealed," "directly attached," and/or "directly joined" to another component, there are no intervening elements present.

Relative terms such as "lower" or "bottom" and "upper" or "top" may be used herein to describe one element's relationship to another element illustrated in the drawings. It will be understood that relative terms are intended to encompass different orientations in addition to the orientation depicted in the drawings. By way of example, if aspects of an exemplary ophthalmic device shown in the drawings are turned over, elements described as being on the "bottom" side of the other elements would then be oriented on the "top" side of the other elements. The term "bottom" can therefore encompass both an orientation of "bottom" and "top" depending on the particular orientation of the apparatus.

Various aspects of an ophthalmic device with an intraocular pressure monitoring system may be illustrated with reference to one or more exemplary embodiments. As used herein, the term "exemplary" means "serving as an example, instance, or illustration," and should not necessarily be construed as preferred or advantageous over other embodiments disclosed herein.

### GLOSSARY

In this description and claims directed to the disclosed invention, various terms may be used for which the following definitions will apply:

Energized: as used herein refers to the state of being able to supply electrical current to or to have electrical energy stored within.

Energy: as used herein refers to the capacity of a physical system to do work. Many uses within this disclosure may relate to the said capacity being able to perform electrical actions in doing work.

Energy Source: as used herein refers to a device or layer that is capable of supplying Energy or placing a logical or electrical device in an energized state.

Energy Harvester: as used herein refers to a device capable of extracting energy from the environment and converting it to electrical energy.

Functionalized: as used herein refers to making a layer or device able to perform a function including for example, energization, activation, or control.

Leakage: as used herein refers to unwanted loss of energy.

Ophthalmic Device: as used herein refers to any device that resides in or on the eye. These devices may provide optical correction, may be cosmetic, or may provide functionality unrelated to the eye. For example, the term lens may refer to a contact lens, intraocular lens, overlay lens, ocular insert, optical insert, or other similar device through which vision is corrected or modified, or through which eye physiology is cosmetically enhanced (e.g. iris color) without impeding vision. Alternatively, the lens may provide non-optic functions such as, for example, monitoring glucose, delivering sound signals and/or administrating medicine. In some embodiments, the preferred lenses of the invention are soft contact lenses are made from silicone elastomers or hydrogels, which include, for example, silicone hydrogels, and fluorohydrogels.

Lithium Ion Cell: as used herein refers to an electrochemical cell where Lithium ions move through the cell to generate electrical energy. This electrochemical cell, typically called a battery, may be reenergized or recharged in its typical forms.

Media Insert: as used herein refers to an encapsulated insert that will be included in an energized ophthalmic device. The energization elements and circuitry may be incorporated in the media insert. The media insert defines the primary purpose of the energized ophthalmic device. For example, in embodiments where the energized ophthalmic device allows the user to adjust the optic power, the media insert may include energization elements that control a liquid meniscus portion in the optical zone. Alternatively, a media insert may be annular so that the optical zone is void of material. In such embodiments, the energized function of the lens may not be optic quality but may be, for example, monitoring glucose, sound delivery, and/or administering medicine.

Micro-Acoustic Element(s): as used herein can refer to a micro acoustic electromechanical system and/or related components that can be used to conduct audible frequencies from the orb of the eye to the inner ear through the bones in the skull. In some embodiments, the micro-acoustic elements can include, for example, a microelectro-mechanical (MEMS) piezoelectric acoustic transducer and/or a condenser acoustic device, energized by an energy source.

Operating Mode: as used herein refers to a high current draw state where the current over a circuit allows the device to perform its primary energized function.

Optical Zone: as used herein refers to an area of an ophthalmic lens through which a wearer of the ophthalmic lens sees.

Power: as used herein refers to work done or energy transferred per unit of time.

Rechargeable or Re-energizable: as used herein refers to a capability of being restored to a state with higher capacity to do work. Many uses within this invention may relate to the capability of being restored with the ability to flow electrical current at a certain rate and for a certain, reestablished period.

Reenergize or Recharge: as used herein refers to restoring to a state with higher capacity to do work. Many uses within this invention may relate to restoring a device to the capability to flow electrical current at a certain rate and for a certain, reestablished period.

Reference: as use herein refers to a circuit which produces an, ideally, fixed and stable voltage or current output suitable for use in other circuits. A reference may be derived from a bandgap, may be compensated for temperature, supply, and process variation, and may be tailored specifically to a particular application-specific integrated circuit (ASIC).

Reset Function: as used herein refers to a self-triggering algorithmic mechanism to set a circuit to a specific predetermined state, including, for example, logic state or an energization state. A reset function may include, for example, a power-on reset circuit, which may work in conjunction with the switching mechanism to ensure proper bring-up of the chip, both on initial connection to the power source and on wakeup from storage mode.

Sleep Mode or Standby Mode: as used herein refers to a low current draw state of an energized device after the switching mechanism has been closed that allows for energy conservation when operating mode is not required.

Stacked: as used herein means to place at least two component layers in proximity to each other such that at least a portion of one surface of one of the layers contacts a first surface of a second layer. In some embodiments, a film, whether for adhesion or other functions may reside between the two layers that are in contact with each other through said film.

Stacked Integrated Component Devices or SIC Devices: as used herein refers to the products of packaging technologies that assemble thin layers of substrates that may contain electrical and electromechanical devices into operative-integrated devices by means of stacking at least a portion of each layer upon each other. The layers may comprise component devices of various types, materials, shapes, and sizes. Furthermore, the layers may be made of various device production technologies to fit and assume various contours.

Storage Mode: as used herein refers to a state of a system comprising electronic components where a power source is supplying or is required to supply a minimal designed load current. This term is not interchangeable with standby mode.

Substrate Insert: as used herein refers to a formable or rigid substrate capable of supporting an energy source within an ophthalmic lens. In some embodiments, the substrate insert also supports one or more components.

Switching Mechanism: as used herein refers to a component integrated with the circuit providing various levels of resistance that may be responsive to an outside stimulus, which is independent of the ophthalmic device.

Recent developments in ophthalmic devices including, for example, contact lenses, have occurred enabling functionalized ophthalmic devices that can be energized. The energized ophthalmic device can comprise the necessary elements to correct and/or enhance the vision of users using embedded micro-electronics. Additional functionality using micro-electronics can include, for example, variable vision correction, tear fluid analysis, audio, and/or visual feedback to the user. In addition to providing audio/visual functionality, the present disclosure provides for an ophthalmic device that includes an intraocular pressure monitoring system. The intraocular pressure monitoring system can include an energized micro-piezoelectric element with a feedback circuit. In some embodiments, the ophthalmic device can be in wireless communication with one or more wireless device(s) and receive signal data that can be used for the determination of an abnormal intraocular pressure and a correlated cause. The wireless device(s) can include, for example, a smart phone device, a tablet, a personal computer, a FOB, an MP3 player, a PDA, and the such.

Currently available glaucoma treatments seek to lower intraocular pressure to preserve visual function of the eye. A combination of medications, including prostaglandin analogs, beta blockers, alpha agonists, and carbonic anhydrase inhibitors can be used to lower the intraocular pressure of a patient's eye. Combinations of these are also available for some patients that require them. Moreover, either the combination or the individual inhibitor are often changed/rotated by the eye care practitioner to reduce side effects and/or ensure efficacy and provide a more effective treatment. These types of treatments reduce a patient's elevated intraocular pressure that left untreated can cause damage to the optic nerve resulting sometimes in blindness.

As previously mentioned, common diurnal factors that patients can be subject to in everyday life vary and can affect the intraocular pressure of a patent and therefore the diagnosis and treatment of glaucoma. According to aspects of the present disclosure, to avoid misdiagnosing a patient due to exercise, fluid intake, caffeine, systemic medications, respiration and heart rate, glycerol consumption, and other everyday medications, and to provide an accurate/effective monitoring of intraocular pressure, a patient may wear an ophthalmic device with intraocular pressure monitoring capabilities.

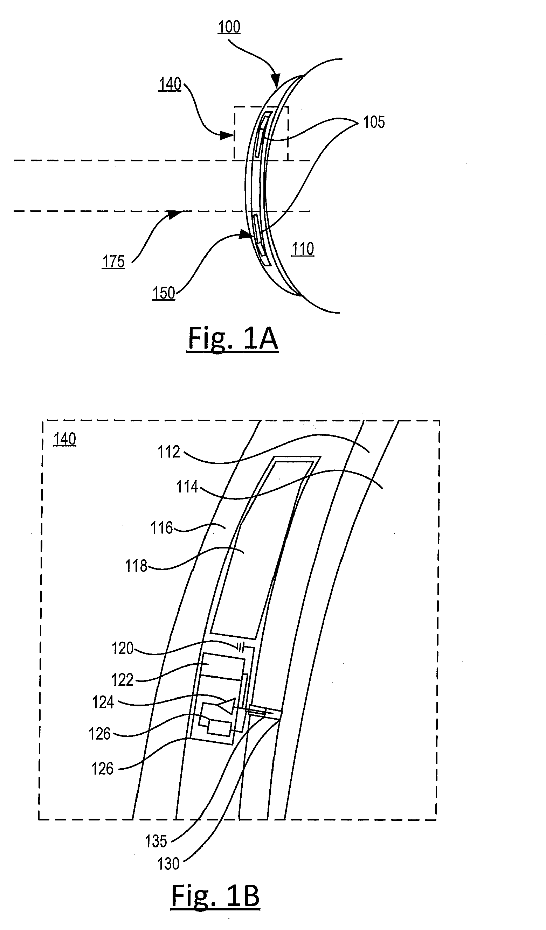

Referring now to Fig. 1A, a diagrammatic cross section representation of a first exemplary energized ophthalmic device 100 comprising both optics and an intraocular pressure monitoring system is depicted. According to some aspects of the present disclosure, the ophthalmic device 100 of the present disclosure may be a contact lens resting on the anterior surface of a patent's eye 110. The contact lens may be a soft hydrogel lens and can include a silicone containing component. A "silicone-containing component" is one that contains at least one [-Si-O-] unit in a monomer, macromer or prepolymer. Preferably, the total Si and attached O are present in the silicone-containing component in an amount greater than about 20 weight percent, and more preferably greater than 30 weight percent of the total molecular weight of the silicone-containing component. Useful silicone-containing components preferably comprise polymerizable functional groups such as acrylate, methacrylate, acrylamide, methacrylamide, vinyl, N-vinyl lactam, N-vinylamide, and styryl functional groups.

Embedded by the hydrogel portion partially or entirely, or in some embodiments placed onto the hydrogel portion, can be a functionalized media insert 150. The media insert 150 can be used to encapsulate electronic elements 105 and in some embodiments energization elements (shown in Fig. 1B). In some embodiments, the electronic elements 105 can preferably be located outside of the optical zone 175, such that the device does not interfere with the patient's sight. System elements 105 may be powered through an external means, energy harvesters, and/or energization elements contained in the ophthalmic device 100. For example, in some embodiments the power may be received using an antenna receiving RF signals that is in communication with the electronic elements 105.

Referring now to Fig. 1B, an enlarged portion 140 of the cross section depicted in Fig. 1A showing aspects of the intraocular pressure monitoring system is depicted. In particular, the enlarged portion 140 illustrates a hydrogel portion 116 of the ophthalmic device 100 resting on ocular fluid 112 on the anterior surface of the eye 110. Ocular fluid 112 can include any one, or a combination of: tear fluid, aqueous humour, vitreous humour, and other interstitial fluids located in the eye. The hydrogel portion 116 encapsulates the media insert 150 which in some embodiments can include energization elements 118, such as a battery and a load, along with the intraocular pressure monitoring system 126.

The intraocular pressure monitoring system 126 can include a wireless communication element 120, such as a RF antenna in connection with a controller 122. The controller 122 can be used to control a piezoelectric-element 130, a pick up 135, and an electronic feedback circuit including an amplifier 124 and a band-pass filter 126 which can all be powered through the Energization elements 118 contained within the media insert 150. The piezoelectric-element 130 and the pick up 135 can resonate a signal and measure the change in the return signal to determine intraocular pressure of the eye 110.

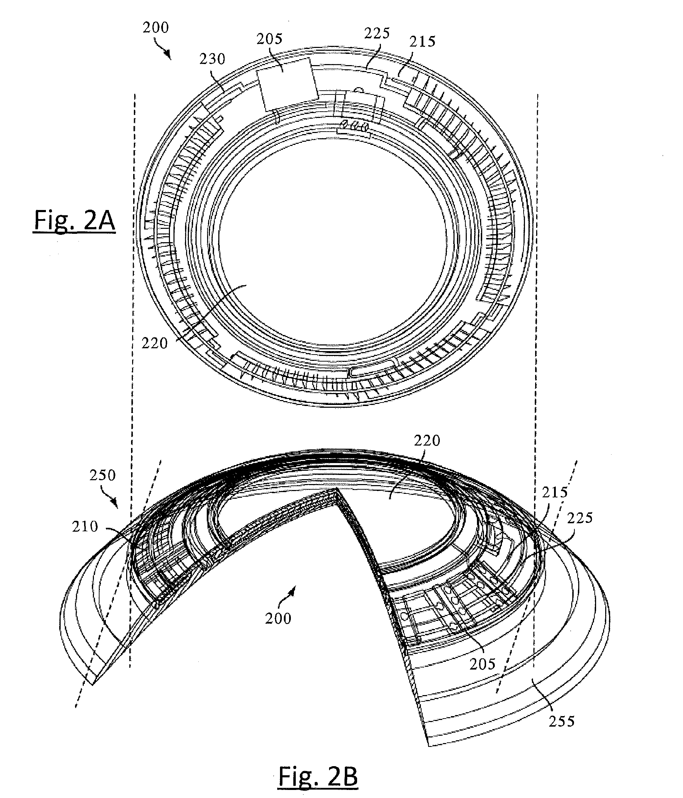

Referring now to Fig. 2A, a diagrammatic representation of the top view of a media insert that may be included as part of another exemplary ophthalmic device comprising both optics and the intraocular pressure monitoring system is depicted. In particular, a top view of an exemplary media insert 200 for an energized ophthalmic device 250 that can include intraocular pressure monitoring system 205 is illustrated. The media insert 200 may comprise an optical zone 220 that may or may not be functional to provide vision correction. Where the energized function of the ophthalmic device is unrelated to vision, the optic zone 220 of the media insert 200 may be void of material. In some embodiments, the media insert 200 may include a portion not in the optical zone 220 comprising a substrate 215 incorporated with energization elements 210 and electronic components 205 which include intraocular pressure monitoring system elements.

In some embodiments, a power source 210, which may be, for example, a battery, and a load 205, which may be, for example, a semiconductor die, may be attached to the substrate 215. Conductive traces 225 and 230 may electrically interconnect the electronic components 205 and the energization elements 210. In some embodiments, the media insert 200 can be fully encapsulated to protect and contain the energization elements 210, traces 225 and 230, and electronic components 205. In some embodiments, the encapsulating material may be semipermeable, for example, to prevent specific substances, such as water, from entering the media insert 200 and to allow specific substances, such as ambient gasses, fluid samples, and/or the byproducts of reactions within energization elements 210, to penetrate and/or escape from the media insert 200.

Referring now to Fig. 2B, a diagrammatic representation of an isometric view of an ophthalmic device including the media insert depicted in Fig. 2A comprising both optics and the intraocular pressure monitoring system is depicted. The media insert 200 may be included in/or on an ophthalmic device 250, which may also comprise a polymeric biocompatible material. The ophthalmic device 250 may include a rigid center, soft skirt design wherein a central rigid optical element comprises the media insert 200. In some specific embodiments, the media insert 200 may be in direct contact with the atmosphere and/or the corneal surface on respective anterior and posterior surfaces, or alternatively, the media insert 200 may be encapsulated in the ophthalmic device 250. The periphery 255 of the ophthalmic device 250 may be a soft skirt material, including, for example, a hydrogel material. The infrastructure of the media insert 200 and the ophthalmic device 250 can provide an environment to monitor the intraocular pressure according to aspects of the present invention. In addition, in the present exemplary ophthalmic device 250, micro-acoustic elements may be placed insider or on a surface of the media insert 200 to transmit audible signals through bone resonance through the skull and to the cochlea. In some embodiments, the audible signals transmitted to the user using the micro-acoustic elements may be transmitted when the intraocular pressure is determined to be outside a predetermined threshold. For example, the audible signal may be a recommended action and/or warning based on levels of the intraocular pressure measured.

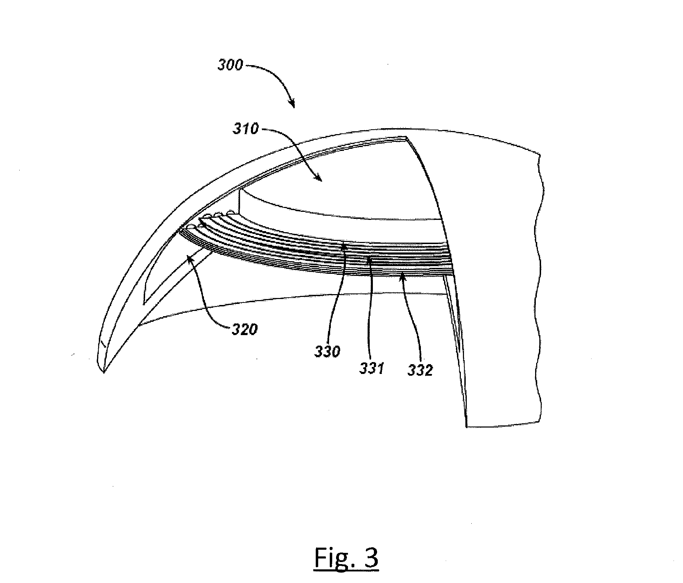

Referring now to Fig. 3, a diagrammatic representation of another exemplary energized ophthalmic device comprising both optics and the intraocular pressure monitoring system is depicted. In particular, a three dimensional cross section representation of an exemplary ophthalmic lens 300 including a functionalized layer media insert 320 configured to include the intraocular pressure monitoring system on one or more of its layers 330, 331, 332 is illustrated. In the present exemplary embodiment, the media insert 320 surrounds the entire periphery of the ophthalmic lens 300. One skilled in the art can understand that the actual media insert 320 may comprise a full annular ring or other shapes that still may reside inside or on the hydrogel portion of the ophthalmic lens 300 and be within the size and geometry constraints presented by the ophthalmic environment of the user.

Layers 330, 331 and 332 are meant to illustrate three of numerous layers that may be found in a media insert 320 formed as a stack of functional layers. In some embodiments, for example, a single layer may include one or more of: active and passive components and portions with structural, electrical or physical properties conducive to a particular purpose including the communication system functions described in the present disclosure. Furthermore, in some embodiments, a layer 330 may include an energy source, such as, one or more of: a battery, a capacitor and a receiver within the layer 330. Item 331 then, in a non-limiting exemplary sense may comprise microcircuitry in a layer that detects actuation signals for the ophthalmic lens 300. In some embodiments, a power regulation layer 332, may be included that is capable of receiving power from external sources, charges the battery layer 330 and controls the use of battery power from layer 330 when the ophthalmic lens 300 is not in a charging environment. The power regulation may also control signals to an exemplary active lens, demonstrated as item 310 in the center annular cutout of the media insert 320.

An energized lens with an embedded media insert 320 may include an energy source, such as an electrochemical cell or battery as the storage means for the energy and in some embodiments, encapsulation, and isolation of the materials comprising the energy source from an environment into which an ophthalmic device is placed. In some embodiments, a media insert 320 can also include a pattern of circuitry, components, and energy sources. Various embodiments may include the media insert 320 locating the pattern of circuitry, components and energy sources around a periphery of an optic zone through which a wearer of an ophthalmic lens would see, while other embodiments may include a pattern of circuitry, components, and energy sources which can be small enough to not adversely affect the sight of the ophthalmic lens wearer and therefore the media insert 320 may locate them within, or exterior to, an optical zone.

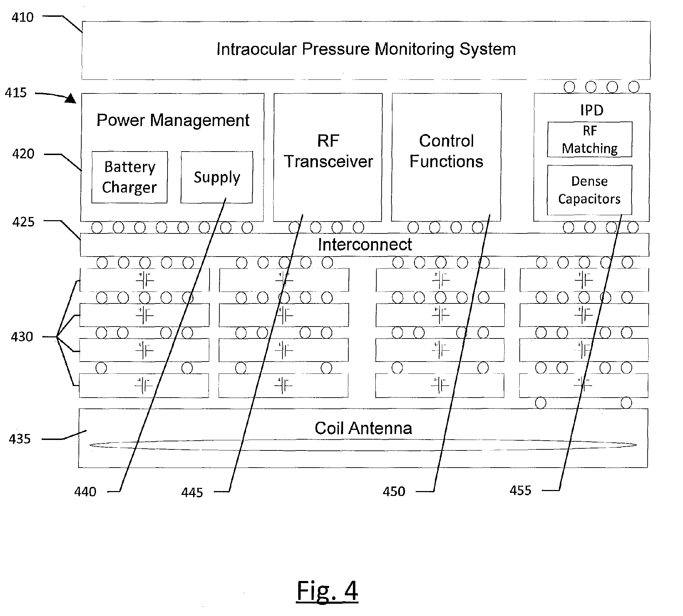

Reference has been made to electronic circuits making up part of the componentry of ophthalmic devices incorporating an intraocular pressure monitoring system. In some embodiments according to aspects of the disclosure, a single and/or multiple discrete electronic devices may be included as discrete chips, for example, in the ophthalmic media inserts. In other embodiments, the energized electronic elements can be included in the media insert in the form of stacked integrated components. Accordingly and referring now to Fig. 4, a schematic diagram of an exemplary cross section of a stacked die integrated components implementing the intraocular pressure monitoring system is depicted. In particular, the media insert may include numerous layers of different types which are encapsulated into contours consistent with the ophthalmic environment that they will occupy. In some embodiments, these media inserts with stacked integrated component layers may assume the entire annular shape of the media insert. Alternatively in some cases, the media insert may be an annulus whereas the stacked integrated components may occupy just a portion of the volume within the entire shape.

As shown in Fig. 4, there may be thin film batteries 430 used to provide energization. In some embodiments, these thin film batteries 430 may comprise one or more of the layers that can be stacked upon each other with multiple components in the layers and interconnections therebetween.

In some embodiments, there may be additional interconnections between two layers that are stacked upon each other. In the state of the art there may be numerous manners to make these interconnections; however, as demonstrated the interconnection may be made through solder ball interconnections between the layers. In some embodiments only these connections may be required; however, in other cases the solder balls may contact other interconnection elements, as for example with a component having through layer vias.

In other layers of the stacked integrated component media insert, a layer 425 may be dedicated for the interconnections two or more of the various components in the interconnect layers. The interconnect layer 425 may contain, vias and routing lines that can pass signals from various components to others. For example, interconnect layer 425 may provide the various battery elements connections to a power management unit 420 that may be present in a technology layer 415. Other components in the technology layer 415 can include, for example, a transceiver 445, control components 450 and the like. In addition, the interconnect layer 425 may function to make connections between components in the technology layer 415 as well as components outside the technology layer 415; as may exist for example in the integrated passive device 455. There may be numerous manners for routing of electrical signals that may be supported by the presence of dedicated interconnect layers such as interconnect layer 425.

In some embodiments, the technology layer 415, like other layer components, may be included as multiple layers as these features represent a diversity of technology options that may be included in media inserts. In some embodiments, one of the layers may include CMOS, BiCMOS, Bipolar, or memory based technologies whereas the other layer may include a different technology. Alternatively, the two layers may represent different technology families within a same overall family; as for example one layer may include electronic elements produced using a 0.5 micron CMOS technology and another layer may include elements produced using a 20 nanometer CMOS technology. It may be apparent that many other combinations of various electronic technology types would be consistent within the art described herein.

In some embodiments, the media insert may include locations for electrical interconnections to components outside the insert. In other examples, however, the media insert may also include an interconnection to external components in a wireless manner. In such cases, the use of antennas in an antenna layer 435 may provide exemplary manners of wireless communication. In many cases, such an antenna layer 435 may be located, for example, on the top or bottom of the stacked integrated component device within the Media Insert.

In some of the embodiments discussed herein, the battery elements 430 may be included as elements in at least one of the stacked layers themselves. It may be noted as well that other embodiments may be possible where the battery elements 430 are located externally to the stacked integrated component layers. Still further diversity in embodiments may derive from the fact that a separate battery or other energization component may also exist within the media insert, or alternatively these separate energization components may also be located externally to the media insert.

Intraocular pressure monitoring system 410 may be included in a stacked integrated component architecture. In some embodiments, the intraocular pressure monitoring system 410 components may be attached as a portion of a layer. In other embodiments, the entire intraocular pressure monitoring system 410 may also comprise a similarly shaped component as the other stacked integrated components.

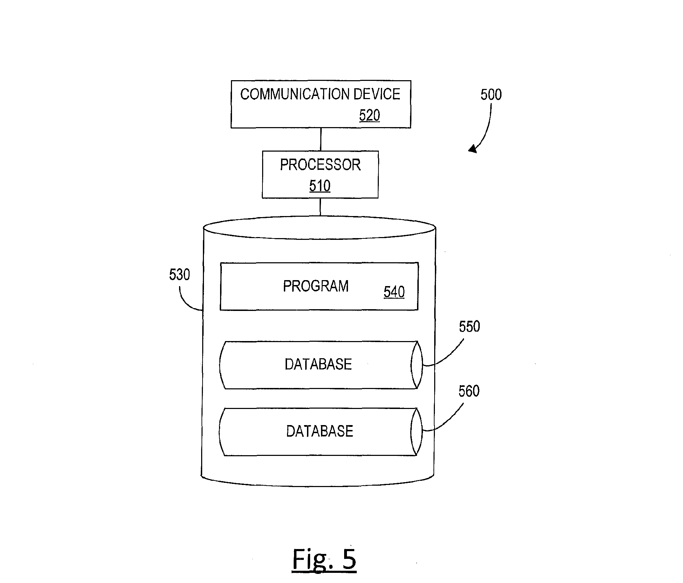

Referring now to Fig. 5 is a schematic diagram of a processor that may be used to implement some aspects of the present disclosure is illustrated. The controller 500 can include one or more processors 510, which may include one or more processor components coupled to a communication device 520. In some embodiments, a controller 500 can be used to transmit energy to the energy source placed in the ophthalmic lens.

The processors 510 are coupled to a communication device configured to communicate energy via a communication channel. The communication device may be used to electronically communicate with components within the media insert, for example. The communication device 520 may also be used to communicate, for example, with one or more controller apparatus or programming/interface device components.

The processor 510 is also in communication with a storage device 530. The storage device 530 may comprise any appropriate information storage device, including combinations of magnetic storage devices, optical storage devices, and/or semiconductor memory devices such as Random Access Memory (RAM) devices and Read Only Memory

(ROM) devices.

The storage device 530 can store a program 540 for controlling the processor 510. The processor 510 performs instructions of a software program 540, and thereby operates in accordance with the present invention. For example, the processor 510 may receive information descriptive of media insert placement, component placement, and the like. The storage device 530 can also store ophthalmic related data in one or more databases 550 and 560. The database may include, for example, predetermined intraocular pressure measurement thresholds, metrology data, and specific control sequences for controlling energy to and from a media insert. The database may also include parameters and controlling algorithms for the control of the intraocular pressure monitoring system that may reside in the ophthalmic device as well as data and/or measured feedback that can result from their action. In some embodiments, that data may be ultimately communicated to/from an external reception device.

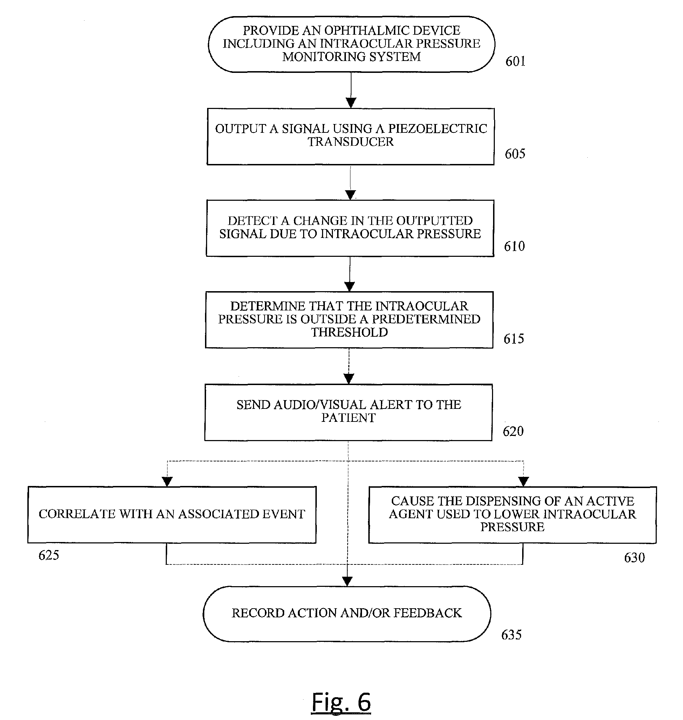

Referring now to Fig. 6, method steps that may be used to implement the intraocular pressure monitoring system of the ophthalmic device is depicted. Beginning at step 601, an ophthalmic device including an intraocular pressure monitoring system is provided to a patient. In some embodiments, the ophthalmic device may include one or two energized contact lenses configured to include a piezoelectric transducer with a feedback circuit used to monitor intraocular pressure, in addition to providing vision correction and/or enhancement.

At step 605, a signal using the piezoelectric transducer can be outputted towards the eye surface. The return signal can be detected and its change after it reflects off the eye surface can be measured to determine the intraocular pressure 610 of a patient's eye. At step 615, when the intraocular pressure is determined to be outside a normal value, between 10 mmHg and 20mm Hg, a signal can be sent to the patient or eye care practitioner at 620. In some embodiments, the signal data may be sent using a wireless device in communication with the ophthalmic device or through an audible signal using micro-acoustic elements included in the ophthalmic device. In some embodiments, the signal may be a visual signal using micro-photonic elements also included in the ophthalmic device. The audible signal may be played in conjunction with a visual signal, e.g., as part of a video clip. Transmission of information with a wireless device can occur wirelessly, for example, via a RF frequency, a local area network (LAN), and/or a private area network (PAN), depending on the communication device and functionality implemented in the ophthalmic device.

At step 625, optionally the signal may be correlated with a specific event imputed by the patient using the interface of the wireless device in communication with the ophthalmic device. For example, a selection from a menu listing activities that can influence intraocular pressure. Activities/events can include but are not limited to the aforementioned factors that can affect intraocular pressure.

In addition, in some embodiments at step 630, the ophthalmic device can include micro fluidic elements configured to dispense a drug/active agent when the intraocular pressure is determined to be abnormal. The drug/active agent can include for example, a combination of medications, including prostaglandin analogs, beta blockers, alpha agonists, and carbonic anhydrase inhibitors are used to lower the intraocular pressure of a patient's eye. In alternative embodiments, the wireless device in communication with the ophthalmic device may include an external drug pump that can dispense the medicine/active agent to lower the intraocular pressure.

Also optionally, at step 635, the action and/or feedback from steps 615-630 can be recorded to improve future analysis, keep a medical record that can be accessed by an eye care practitioner, and/or tailor the intraocular pressure monitoring system to the particular patient. In some embodiments, these recorded actions/records can also be sent/stored using the wireless device. As previously mentioned, the wireless device can include one or more of: a smart phone, tablet, personal computer, television, drug pump, etc. Transmission of information between them can occur wirelessly, for example, via an RF frequency, a local area network (LAN), and/or a private area network (PAN), depending on the communication device and functionality implemented in the ophthalmic device.

The many features and advantages of the invention are apparent from the detailed specification, and thus, it is intended by the appended claims to cover all such features and advantages of the invention which fall within the true spirit and scope of the invention. Further, because numerous modifications and variations will readily occur to those skilled in the art, it is not desired to limit the invention to the exact construction and operation illustrated and described, and accordingly, all suitable modifications and equivalents may be resorted to, falling within the scope of the invention.

A non-exhaustive list of aspects of the disclosure is set out in the following numbered clauses:
Clause 1. An ophthalmic device with an intraocular pressure monitoring system, the ophthalmic device comprising:
   a media insert comprising a front curve arcuate surface and a back curve arcuate surface, wherein the front curve arcuate surface and the back curve arcuate surface form a cavity capable of containing an energy source dimensioned to conform to an area within the cavity, wherein the energy source is in electrical connection and capable of energizing a micro-piezoelectric element with an electronic feedback circuit and a controller, the controller comprising a computer processor in digital communication with a digital media storage device and wherein the digital media storage device stores software code;
   a transmitter in logical communication with the processor and also in logical communication with a communication network, wherein the software is executable upon demand and operative with the processor to:
      output a signal using the micro-piezoelectric element;
      detect the change of the outputted signal using the electronic feedback circuit; and
      determine the intraocular pressure of a user's eye using the detected change of said outputted signal.
Clause 2. The ophthalmic device of clause 1, additionally comprising: a radio frequency antenna in connection with the communication network and capable of transmitting data with a wireless device.
Clause 3. The ophthalmic device of clause2, wherein the software is additionally operative with the processor to:
   send a signal to the wireless device when the determined intraocular pressure is outside a predetermined threshold.
Clause 4. The ophthalmic device of clause 2, additionally comprising: a photon emitter element in connection with the communication network and capable of providing a visual signal to the user when the determined intraocular pressure is outside a predetermined threshold.
Clause 5. The ophthalmic device of clause 2, additionally comprising: a micro-electromechanical transducer capable of transmitting an audible signal to the user when the determined intraocular pressure is outside a predetermined threshold.
Clause 6. The ophthalmic device of clause 2, wherein the software is operative with the processor to:
   transmit, through the antenna, to a drug dispensing device a signal to dispense an active agent when the determined intraocular pressure is outside a predetermined threshold.
Clause 7. The ophthalmic device of clause 6, wherein the active agent is a medication including one or more active agents of: prostaglandin analogs, beta blockers, alpha agonists, and carbonic anhydrase inhibitors.
Clause 8. The ophthalmic device of clause 1, additionally comprising: one or more reservoir(s) capable of containing a volume of an active agent used to treat glaucoma.
Clause 9. The ophthalmic device of clause 8, wherein the software is operative with the processor to:
   dispense, from the one or more reservoir(s), an active agent when the determined intraocular pressure is outside a predetermined threshold.
Clause 10. The ophthalmic device of clause 1, wherein the software is operative with the processor to:
   correlate a change of intraocular pressure with an associated event.
Clause 11. The ophthalmic device of clause 1, wherein the software is operative with the processor to:
   record one or both an action or a feedback from the Ophthalmic Device after the intraocular pressure is determined to be outside a predetermined threshold.
Clause 12. The ophthalmic device of clause 1, wherein the energy source is fabricated using stacked integrated component device packaging technologies.
Clause 13. A method of monitoring the intraocular pressure of a patient's eye, comprising:
   providing an ophthalmic device with a intraocular pressure monitoring system comprising an energy source in electrical connection and capable of energizing a micro-piezoelectric element with an electronic feedback circuit and a controller comprising a computer processor, a digital media storage device, a transmitter in logical communication with the processor and also in logical communication with a communication network;
   outputting a signal using the micro-piezoelectric element:
      receiving and measuring a return signal resulting from the outputted signal with the electronic feedback circuit; and
      determining the intraocular pressure of a user's eye using the measured return signal.
Clause 14. The method of clause 13, additionally comprising:
   sending a signal to a wireless device in wireless communication with the processor of the ophthalmic device, wherein the signal corresponds to the intraocular pressure determination.
Clause 15. The method of clause 13, additionally comprising:
   sending a visual alert to the user through a photon emitter element forming part of the ophthalmic device and in connection with the communication network when the intraocular pressure is outside a predetermined threshold.
Clause 16. The method of clause 13, additionally comprising:
   sending an audible signal to the user through an electromechanical transducer forming part of the ophthalmic device and in connection with communication network when the intraocular pressure is outside a predetermined threshold.
Clause 17. The method of clause 14, wherein the wireless device is one or more of a cellular device, a biomedical device, a drug dispensing device, a tablet, and a personal computer.
Clause 18. The method of clause 13, additionally comprising:
   recording the intraocular pressure determination as part of the patient's medical history.
Clause 19. The method of clause 18, additionally comprising:
   dispensing an active agent when the intraocular pressure determination falls outside a predetermined threshold.
Clause 20. A method of monitoring intraocular pressure of a patient's eye, comprising:
   providing an ophthalmic device with an intraocular pressure monitoring system comprising an energy source in electrical connection and capable of energizing a micro-piezoelectric element with an electronic feedback circuit and a controller comprising a computer processor, a digital media storage device, a transmitter in logical communication with the processor and also in logical communication with a communication network;
   outputting a signal using the micro-piezoelectric element:
      receiving and measuring a return signal resulting from the outputted signal with the electronic feedback circuit;
      determining the intraocular pressure of a user's eye using the measured return signal; and
      recording the determined intraocular pressure in the digital media storage device.

## Claims

1. An ophthalmic device with an intraocular pressure monitoring system, the ophthalmic device comprising:
a media insert comprising a front curve arcuate surface and a back curve arcuate surface, wherein the front curve arcuate surface and the back curve arcuate surface form a cavity capable of containing an energy source dimensioned to conform to an area within the cavity, wherein the energy source is in electrical connection and capable of energizing a micro-piezoelectric element with an electronic feedback circuit and a controller, the controller comprising a computer processor in digital communication with a digital media storage device and wherein the digital media storage device stores software code;
a transmitter in logical communication with the processor and also in logical communication with a communication network, wherein the software is executable upon demand and operative with the processor to:
output a signal using the micro-piezoelectric element;
detect the change of the outputted signal using the electronic feedback circuit; and
determine the intraocular pressure of a user's eye using the detected change of said outputted signal.

2. The ophthalmic device of claim 1, additionally comprising: a radio frequency antenna in connection with the communication network and capable of transmitting data with a wireless device.

3. The ophthalmic device of claim 2, wherein the software is additionally operative with the processor to:
send a signal to the wireless device when the determined intraocular pressure is outside a predetermined threshold.

4. The ophthalmic device of any of claims 1 to 3, additionally comprising: a photon emitter element in connection with the communication network and capable of providing a visual signal to the user when the determined intraocular pressure is outside a predetermined threshold.

5. The ophthalmic device of any of claims 1 to 4, additionally comprising: a micro-electromechanical transducer capable of transmitting an audible signal to the user when the determined intraocular pressure is outside a predetermined threshold.

6. The ophthalmic device of claim 2 or claim 3, wherein the software is operative with the processor to:
transmit, through the antenna, to a drug dispensing device a signal to dispense an active agent when the determined intraocular pressure is outside a predetermined threshold, wherein preferably the active agent is a medication including one or more active agents of: prostaglandin analogs, beta blockers, alpha agonists, and carbonic anhydrase inhibitors.

7. The ophthalmic device of any of the preceding claims, additionally comprising:
one or more reservoir(s) capable of containing a volume of an active agent used to treat glaucoma.

8. The ophthalmic device of claim 7, wherein the software is operative with the processor to:
dispense, from the one or more reservoir(s), an active agent when the determined intraocular pressure is outside a predetermined threshold.

9. The ophthalmic device of any of the preceding claims, wherein the software is operative with the processor to:
correlate a change of intraocular pressure with an associated event; and/or
record one or both an action or a feedback from the Ophthalmic Device after the intraocular pressure is determined to be outside a predetermined threshold.

10. The ophthalmic device of any of the preceding claims, wherein the energy source is fabricated using stacked integrated component device packaging technologies.

11. A method of monitoring the intraocular pressure of a patient's eye, comprising:
providing an ophthalmic device with a intraocular pressure monitoring system comprising an energy source in electrical connection and capable of energizing a micro-piezoelectric element with an electronic feedback circuit and a controller comprising a computer processor, a digital media storage device, a transmitter in logical communication with the processor and also in logical communication with a communication network;
outputting a signal using the micro-piezoelectric element:
receiving and measuring a return signal resulting from the outputted signal with the electronic feedback circuit; and
determining the intraocular pressure of a user's eye using the measured return signal.

12. The method of claim 11, additionally comprising:
sending a signal to a wireless device in wireless communication with the processor of the ophthalmic device, wherein the signal corresponds to the intraocular pressure determination.

13. The method of claim 12, wherein the wireless device is one or more of a cellular device, a biomedical device, a drug dispensing device, a tablet, and a personal computer.

14. The method of any of claims 11 to 13, additionally comprising:
sending a visual alert to the user through a photon emitter element forming part of the ophthalmic device and in connection with the communication network when the intraocular pressure is outside a predetermined threshold.

15. The method of any of claims 11 to 14, additionally comprising:
sending an audible signal to the user through an electromechanical transducer forming part of the ophthalmic device and in connection with communication network when the intraocular pressure is outside a predetermined threshold.

16. The method of any of claims 11 to 15, additionally comprising:
recording the intraocular pressure determination as part of the patient's medical history.

17. The method of claim 16, additionally comprising:
dispensing an active agent when the intraocular pressure determination falls outside a predetermined threshold.

18. A method of monitoring intraocular pressure of a patient's eye, comprising:
providing an ophthalmic device with an intraocular pressure monitoring system comprising an energy source in electrical connection and capable of energizing a micro-piezoelectric element with an electronic feedback circuit and a controller comprising a computer processor, a digital media storage device, a transmitter in logical communication with the processor and also in logical communication with a communication network;
outputting a signal using the micro-piezoelectric element:
receiving and measuring a return signal resulting from the outputted signal with the electronic feedback circuit;
determining the intraocular pressure of a user's eye using the measured return signal; and
recording the determined intraocular pressure in the digital media storage device.
